# EUROPEAN PATENT APPLICATION

(11) **EP 4 830 635 A1**
(43) Date of publication of application: **28.10.2026**
(21) Application number: 24906095.5
(22) Date of filing: 04.12.2024
(51) Int. Cl.: A61B 6/03

(54) **STATIC CT DEVICE AND CT EXAMINATION METHOD**

(30) Priority: 21.12.2023 CN 202311771056
(71) Applicant: Nuctech Company Limited, TongFang Building Shuangqinglu Haidian District Beijing 100084 (CN); Tsinghua University, Beijing 100084 (CN)
(72) Inventor: CHEN, Zhiqiang, Beijing 100084 (CN); ZHANG, Li, Beijing 100084 (CN); LI, Xinbin, Beijing 100084 (CN); WANG, Zhentian, Beijing 100084 (CN); HUANG, Qingping, Beijing 100084 (CN); SUN, Yunda, Beijing 100084 (CN); SHEN, Le, Beijing 100084 (CN); FENG, Bo, Beijing 100084 (CN)
(74) Representative: Winter, Brandl - Partnerschaft mbB
(86) International application number: PCT/CN2024/136697
(87) International publication number: WO 2025/130611

(57) **Abstract**

A static CT apparatus and a CT inspection method. The apparatus includes one or more inspection assemblies, each inspection assembly including: a distributed multi-point source (S) configured to emit radiation beams; a detector (DT) disposed opposite to the distributed multi-point source to receive the radiation beams emitted by the distributed multi-point source, where the distributed multi-point source and the detector define an inspection channel; and a grating (G0, G1, G2) arranged in the inspection channel and between the distributed multi-point source and the detector. The grating is provided with a periodic structure configured to diffract the radiation beams to generate an interference fringe.

## Description

This application claims the benefit of the priority to Chinese Patent Application No. 202311771056.2 filed on December 21, 2023, the entire contents of which are incorporated herein by reference.

### TECHNICAL FIELD

The present disclosure relates to a field of detection technology. Specifically, the present disclosure relates to a static CT apparatus.

### BACKGROUND

Related X-ray Computed Tomography (CT) imaging apparatuses are usually implemented based on existing X-ray absorption imaging. Their core components include the X-ray source and the detector, which may obtain absorption images reflecting attenuation characteristics of X-rays passing through substances. Such absorption images exhibit strong discriminating capability for high-density substances having strong X-ray absorption capability, and provide excellent imaging effects for substances such as metals, bones, and the like. Therefore, absorption images have been widely used in security inspection fields for identifying contraband and in medical fields for diagnosing bone diseases. However, for substances composed of light elements (such as carbon, hydrogen, oxygen, nitrogen, etc.), the signals in absorption images are weak, resulting in significant limitations. For example, in the field of security inspection, it is difficult to observe contraband such as drugs shielded by metal, and in the medical field, it is difficult to observe pathological tissues in the lungs. In summary, in order to improve the effectiveness of security inspection and medical diagnosis, new technologies and new products that help enhance the image discriminating capability for weakly absorbing substances may be explored.

### SUMMARY

According to an aspect of the present disclosure, a static CT apparatus is provided, including one or more inspection assemblies, where each of the one or more inspection assemblies includes: a distributed multi-point source, including a plurality of point sources arranged in an array, and configured to emit radiation beams; a detector disposed opposite to the distributed multi-point source to receive the radiation beams emitted by the distributed multi-point source, where the distributed multi-point source and the detector define an inspection channel; and a grating arranged between the distributed multi-point source and the detector, where the grating is provided with a periodic structure, and the periodic structure is configured to diffract the radiation beams to generate an interference fringe.

In an embodiment, the one or more inspection assemblies are arranged in different sections along an extension direction of the inspection channel.

In an embodiment, in a transverse direction of the extension direction of the inspection channel, the one or more inspection assemblies emit radiation beams toward the inspection channel from different viewing angles in a circumferential direction of the inspection channel relative to an object under inspection.

In an embodiment, the one or more inspection assemblies include four inspection assemblies, respective distributed multi-point sources of the four inspection assemblies are respectively arranged on a top side of the inspection channel, a bottom side of the inspection channel, a left side of the inspection channel, and a right side of the inspection channel, so as to emit radiation beams toward the inspection channel respectively from the top side, the bottom side, the left side, and the right side of the inspection channel along a transverse cross-section of the extension direction of the inspection channel.

In an embodiment, the one or more inspection assemblies are arranged in a same section along the extension direction of the inspection channel, and in each of the one or more inspection assemblies, the distributed multi-point source and the detector are oppositely arranged along a transverse direction of the extension direction of the inspection channel, and the one or more inspection assemblies are configured to emit radiation beams toward the inspection channel from different viewing angles in a circumferential direction of the inspection channel.

In an embodiment, the one or more inspection assemblies are arranged such that directions in which respective distributed multi-point sources of the inspection assemblies emit radiation beams toward the detectors are offset from each other.

In an embodiment, the one or more inspection assemblies include four inspection assemblies, respective distributed multi-point sources of the four inspection assemblies are respectively arranged on a top side of the inspection channel, a bottom side of the inspection channel, a left side of the inspection channel, and a right side of the inspection channel, so as to emit radiation beams toward the inspection channel respectively from the top side, the bottom side, the left side, and the right side of the inspection channel along the transverse direction of the extension direction of the inspection channel.

In an embodiment, the one or more inspection assemblies are configured to operate simultaneously or in a time-sequential manner.

In an embodiment, in each of the one or more inspection assemblies, the plurality of point sources of the distributed multi-point source are configured to emit radiation beams in a time-sequential manner.

In an embodiment, in at least one of the one or more inspection assemblies, the gratings include: a first grating arranged adjacent to and parallel to the distributed multi-point source and spaced apart from the distributed multi-point source by a first distance; and/or a second grating arranged adjacent to and parallel to the distributed multi-point source and spaced apart from the distributed multi-point source by a second distance different from the first distance; or a second grating arranged adjacent to and parallel to the detector and spaced apart from the distributed multi-point source by a third distance; and/or a third grating arranged adjacent to and parallel to the detector and spaced apart from the distributed multi-point source by a fourth distance different from the third distance.

In an embodiment, in at least one of the one or more inspection assemblies, the gratings include a first grating, a second grating, and a third grating, and the first grating, the second grating, and the third grating are parallel to each other.

In an aspect of the present disclosure, a CT inspection method implemented by the static CT apparatus described above is provided, the method including: operating the one or more inspection assemblies simultaneously or in a time-sequential manner to inspect an object under inspection from different viewing angles of the inspection channel, where the plurality of point sources of the distributed multi-point source of each inspection assembly emit the radiation beams in a flying-spot manner so as to irradiate the object under inspection from different angles.

It should be understood that the content described in this section is not intended to identify key or essential features of embodiments of the present disclosure, nor is it intended to limit the scope of the present disclosure. Other features of the present disclosure will become readily understandable through the following description.

### BRIEF DESCRIPTION OF THE DRAWINGS

The accompanying drawings are provided for better understanding of the present solution and do not constitute any limitations to the present disclosure, in which:
FIG. 1 shows various arrangements of gratings in an imaging apparatus;
FIG. 2 shows an arrangement of one or more inspection assemblies according to an embodiment of the present disclosure;
FIG. 3 shows an arrangement of one or more inspection assemblies according to an embodiment of the present disclosure, in which the one or more inspection assemblies are arranged in a same section;
FIG. 4 shows a structure of an inspection assembly according to an embodiment of the present aspect, including three gratings G0, G1, and G2, and further shows an example of a grating;
FIG. 5 shows two-dimensional images for absorption *T* (a), phase Φ (b), and small-angle scattering *D* (c), as well as corresponding XY-plane slice images of three-dimensional images for linear attenuation coefficient *µ* (d), refractive index decrement *δ* (e), and linear diffusion coefficient *ζ* (f) reconstructed using an FBP algorithm from 360 projections acquired over one rotation (corresponding to Equation (3) of the technical disclosure document); and
FIG. 6 shows stained histological microscopic images (a1 to a6) of human breast specimens 1 to 6, as well as corresponding XZ-plane slice images of reconstructed three-dimensional images for linear attenuation coefficient *µ* (b1 to b6) and XZ-plane slice images of reconstructed three-dimensional images for refractive index decrement *δ* (c1 to c6).

### DETAILED DESCRIPTION OF EMBODIMENTS

To more clearly describe the objectives, technical solutions, and advantages of the present disclosure, embodiments of the present disclosure will be described in detail below with reference to the accompanying drawings. It should be understood that the following description of embodiments is intended to explain and illustrate the general concept of the present disclosure and should not be construed as limiting the present disclosure. In the specification and the accompanying drawings, the same or similar reference numerals denote the same or similar components or members. For purposes of clarity, the drawings are not necessarily drawn to scale, and certain well-known components and structures may be omitted from the drawings.

Unless otherwise defined, technical or scientific terms used herein shall have the meanings commonly understood by a person having ordinary skill in the art to which the present disclosure pertains. Terms such as "first", "second", and similar expressions used herein do not indicate any order, quantity, or importance, but are merely used to distinguish different components. Terms "a" and "an" do not exclude the presence of multiple items. Terms such as "comprising", "including", or similar expressions indicate that the element or item preceding the word encompasses the element or item listed thereafter as well as equivalents thereof, but does not exclude additional elements or items. Terms such as "connected", "coupled", or similar expressions are not limited to physical or mechanical connections and may include electrical connections, whether direct or indirect. Terms such as "upper", "lower", "left", "right", "top", "bottom", and the like are used only to indicate relative positional relationships, and such relative positional relationships may change when the absolute position of the described object changes. When an element such as a layer, film, region, or substrate is described as being arranged "on" or "under" another element, the element may be directly on or under the other element, or one or more intermediate elements may be present therebetween.

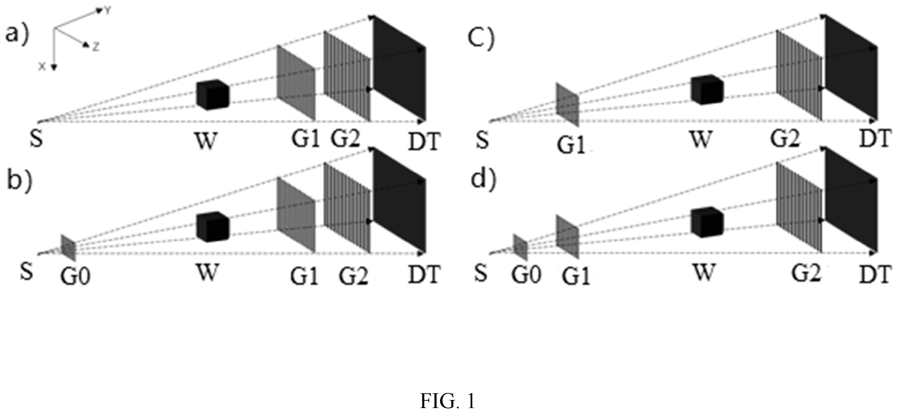

In (a), (b), (c), and (d) of FIG. 1, various grating imaging arrangements are shown, and G1, G2, and G3 represent three gratings, which are arranged between a source and an object under inspection or between an object under inspection and a detector as shown in (a), (b), (c), and (d) of FIG. 1.

In grating imaging technology, both the wave nature and the particle nature of X-rays may be utilized to acquire multiple characteristic information including three types of information, namely absorption information, phase information, and small-angle scattering information, where the absorption information and the phase information respectively correspond to an imaginary part *β* and a real part *δ* of a complex refractive index n. $n = 1 - δ + iβ$ (1) where a linear attenuation coefficient µ has a linear relationship with the imaginary part β (λ represents a wavelength of the X-rays). $μ = \frac{4 πβ}{λ}$ (2)

The three types of characteristic information of absorption *T,* phase Φ, and small-angle scattering *D* may be respectively regarded as integrals of the linear attenuation coefficient µ, the refractive index decrement *δ*, and the linear diffusion coefficient *ζ* (where *L* represents a propagation path of X-rays, and *c*_{Φ} and *c_{D}* are both constant values related to the imaging system). $\left\{\begin{matrix}T = exp \left(-{\int }_{L} μ \left(\vec{r}\right) d \vec{r}\right) \\ Φ = {c}_{Φ} ⋅ \frac{\partial }{\partial x} \left({\int }_{L} δ \left(\vec{r}\right) d \vec{r}\right) \\ D = {c}_{D} ⋅ exp \left(-{\int }_{L} ζ \left(\vec{r}\right) d \vec{r}\right)\end{matrix}\right.$ (3)

Considering that X-rays generally exhibit an energy spectrum distribution, and assuming that a normalized energy spectrum distribution is represented by *S*(E), Equation (3) may be expressed as: $\left\{\begin{matrix}T = {\int }_{{E}_{0}}^{{E}_{1}} S \left(E\right) ⋅ exp \left(-{\int }_{L} μ \left(\vec{r}, E\right) d \vec{r}\right) dE \\ Φ = {c}_{Φ} ⋅ {\int }_{{E}_{0}}^{{E}_{1}} S \left(E\right) ⋅ \frac{\partial }{\partial x} \left({\int }_{L} δ \left(\vec{r}, E\right) d \vec{r}\right) dE \\ D = {c}_{D} ⋅ {\int }_{{E}_{0}}^{{E}_{1}} S \left(E\right) ⋅ exp \left(-{\int }_{L} ζ \left(\vec{r}, E\right) d \vec{r}\right) dE\end{matrix}\right.$ (4)

In (a) of FIG. 1, the grating G1 may function to form a self-imaging fringe pattern in a downstream optical path. When X-rays interact with a substance, the self-imaging fringe pattern may undergo local distortion and deformation. Such deformation occurs on a micrometer scale or even a sub-micrometer scale, which is beyond the resolution that is achieved by ordinary detectors. Therefore, grating G2 amplifies the signal. By observing variations in the intensity of the detector signal, three characteristic images of absorption, phase, and small-angle scattering may be extracted. When a focal spot of the X-ray source is sufficiently small (e.g., in a synchrotron radiation source or a micro-focus X-ray source), only gratings G1 and G2 are required, as shown in (a) and (c) of FIG. 1.

When the focal spot of the X-ray source has a large size (e.g., in an existing X-ray source), the resulting blurring effect may deteriorate the imaging effect. Therefore, a grating G0 may be added downstream of the X-ray source. Due to the presence of grating G0, the radiation beams emitted by the X-ray source may be converted into a series of partially coherent micrometer-scale linear radiation beams, as shown in (b) and (d) of FIG. 1.

An object under inspection W may be positioned between grating G0 and grating G1 (as shown in (b) of FIG. 1), or between grating G1 and grating G2 (as shown in (c) and (d) of FIG. 1).

When the object under inspection W is positioned between gratings G0 and G1, as shown in (b) of FIG. 1, in this configuration, stronger phase signals and small-angle scattering signals may be obtained, resulting in higher imaging contrast and sensitivity. However, this configuration also has certain disadvantages, that is, grating G1 has a large area and incurs higher cost. In addition, X-rays transmitted through the object under inspection W may be blocked by gratings G1 and G2 before being received by the detector, resulting in low X-ray utilization efficiency.

When the object under inspection W is positioned between gratings G1 and G2, as shown in (c) and (d) of FIG. 1, in this configuration, grating G1 is located close to the light source and may have a small area and low cost. Furthermore, X-rays transmitted through the object under inspection W are blocked only by grating G2, resulting in higher X-ray utilization efficiency. However, the phase signals and small-angle scattering signals obtained in this configuration are somewhat weaker. Nevertheless, for certain strongly scattering substances, this is less likely to cause detector signal saturation.

The absorption image (the above T) reflects attenuation characteristics of the substance with respect to X-rays, corresponding to the imaginary part *β* of the complex refractive index of the substance for X-rays (complex refractive index *n* = 1 - *δ* + *iβ*). The phase image reflects refraction effects of the substance on X-rays, corresponding to the real part *δ* of the complex refractive index of the substance for X-rays. For weakly absorbing substances, the phase image advantageously provides higher contrast than the absorption image, and *δ* may be higher than *β* by 2 to 3 orders of magnitude. Accordingly, weakly absorbing substances may be displayed more clearly through the phase image. The small-angle scattering image reflects a mesoscopic structural information of the substance and is highly sensitive to differences at micrometer scale or sub-micrometer scale, such as porosity and multi-fiber structures. Accordingly, the small-angle scattering image may break through the resolution limitations of absorption imaging. The phase image and the small-angle scattering image are valuable supplements to the absorption image, which may effectively improve the overall imaging effect and resolving capability for the object under inspection W, and enhance the adaptability and accuracy of the inspection process for different substances.

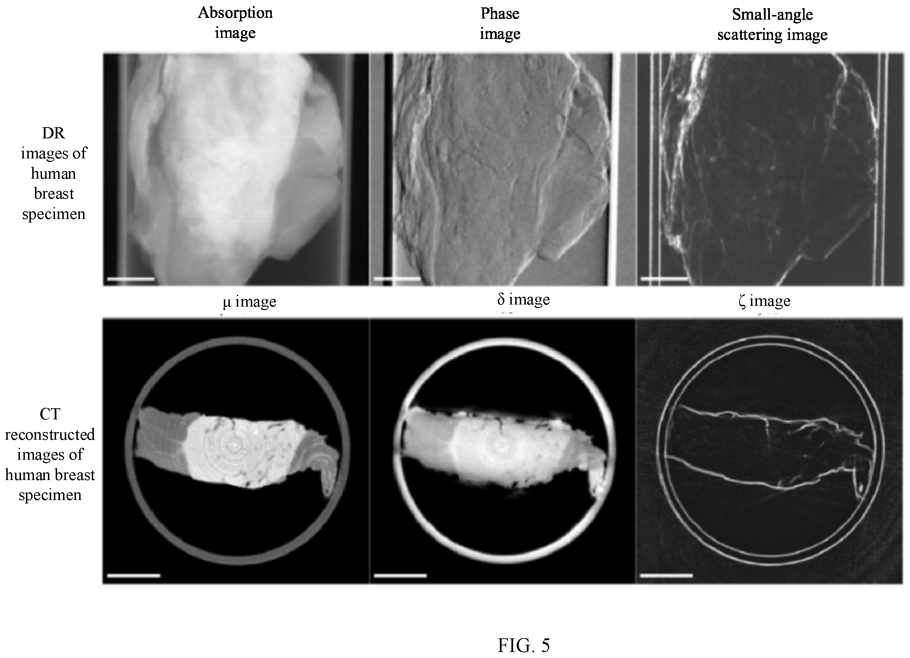

FIG. 5 shows two-dimensional images for absorption *T* (a), phase Φ (b), and small-angle scattering *D* (c), as well as corresponding XY-plane slice images of three-dimensional images for linear attenuation coefficient *µ* (d), refractive index decrement *δ* (e), and linear diffusion coefficient *ζ* (f) reconstructed using FBP algorithm from 360 projections acquired over one rotation (corresponding to the Equation (3) of the technical disclosure document). The absorption image (corresponding to the linear attenuation coefficient *µ*) reflects attenuation characteristics of the substance. The phase image (corresponding to the refractive index decrement *δ* in the complex refractive index) provides higher contrast for weakly absorbing substances and reflects a texture information of internal and external structures of the object under inspection W. The small-angle scattering image (corresponding to the linear diffusion coefficient *ζ*) reflects the mesoscopic structural information of the substance and reflects microscopic structures such as porosity and multi-fiber structures at the micrometer/sub-micrometer scale.

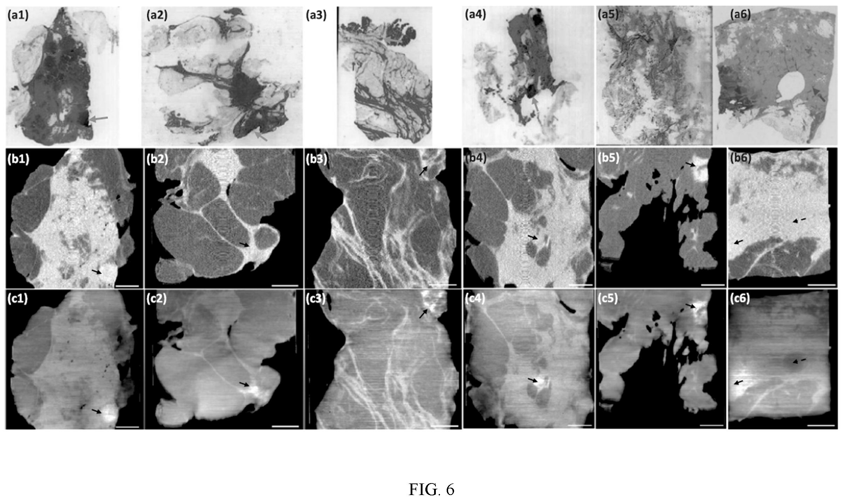

FIG. 6 shows stained histological microscopic images (al to a6) of human breast specimens 1 to 6, as well as corresponding XZ-plane slice images of reconstructed three-dimensional images for linear attenuation coefficient *µ* (b1 to b6), and XZ-plane slice images of reconstructed three-dimensional images for refractive index decrement *δ* (c1 to c6).

In specimens 1, 2, 4, and 5, the arrows indicate tumor lesions (darkened regions); in specimen 3, the arrow indicates pectoral muscle tissue; in specimen 6, the arrow indicates a necrotic region (darkened region).

In all specimen images, fat tissues may be readily distinguished. However, in the reconstructed image with phase information, a tumor region may be more clearly distinguished from surrounding fibroglandular tissues, and a necrotic region may also be distinguished. Grating imaging advantageously compensates for the limitations of grating-free two-dimensional absorption imaging. A combination of the two enables a more comprehensive analysis of the specimen.

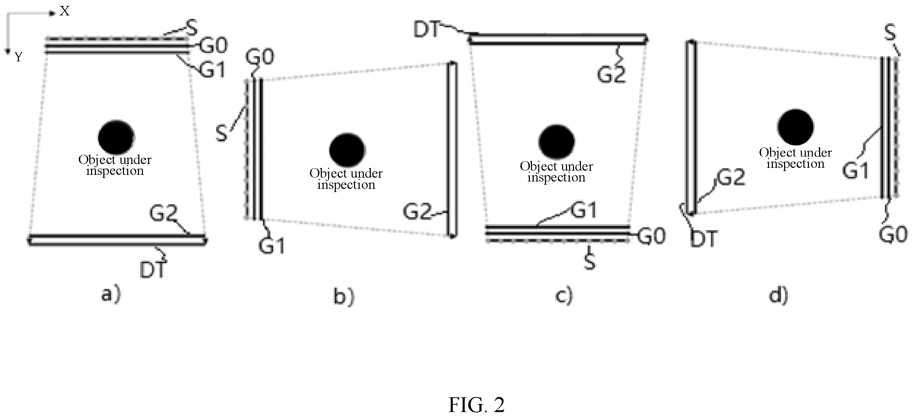

As shown in FIG. 2, according to an embodiment of the present disclosure, a static CT apparatus includes one or more inspection assemblies, where each of the one or more inspection assemblies includes: a distributed multi-point source S, including a plurality of point sources arranged in an array, and used to emit radiation beams; a detector DT disposed opposite to the distributed multi-point source S to receive the radiation beams emitted by the distributed multi-point source S, where the distributed multi-point source S and the detector DT define an inspection channel; and a grating arranged between the distributed multi-point source S and the detector DT. In such embodiments, the grating is provided with a periodic structure used to diffract the radiation beams to generate one or more interference fringes. In embodiments of the present disclosure, the one or more inspection assemblies may include one, two, three, four, or other numbers of inspection assemblies, all of which may perform static CT imaging. Each inspection assembly includes a distributed multi-point source S and a detector DT. The distributed multi-point source S includes a plurality of point sources, for example, five point sources, seven point sources, or the like. The point sources may be arranged in a single row or a single column, for example, along a straight line, or along a curve. Alternatively, the point sources may be arranged in two rows or two columns or in other forms. The point sources may emit radiation beams in a time-sequential manner. The radiation beams may be conical radiation beams, fan beams, pencil beams, or the like. The morphology of the radiation beams may be selected as needed. For example, an appropriate collimator may be provided to shape the radiation beams emitted by each point source. That is, in the present disclosure, the distributed multi-point source S may include an appropriate collimator. FIG. 2 shows that each inspection assembly includes gratings G0, G1, and G2. However, it should be understood that in other embodiments, the one or more inspection assemblies may include only one of grating G0, grating G1, or grating G2, which means any one thereof arranged at the corresponding position as shown. Furthermore, in some embodiments, the one or more inspection assemblies may include different gratings. For example, one inspection assembly may include grating G1, another inspection assembly may include grating G0, and yet another inspection assembly may include grating G2. The various embodiments will not be described in detail herein, and those skilled in the art may determine the configuration as needed according to the present disclosure.

In an embodiment, the static CT apparatus includes only one inspection assembly. The inspection assembly includes: a distributed multi-point source S, including a plurality of point sources arranged in an array, and used to emit radiation beams; a detector DT disposed opposite to the distributed multi-point source S to receive the radiation beams emitted by the distributed multi-point source S, where the distributed multi-point source S and the detector DT define an inspection channel; and a grating arranged between the distributed multi-point source S and the detector DT. Due to the arrangement of the multi-point source, the plurality of point sources may irradiate the object under inspection from multiple angles. Accordingly, without moving the object under inspection, multi-angle imaging and synthesis of at least one tomographic slice of the object under inspection may be performed to construct a three-dimensional image of the tomographic slice.

In an embodiment, a plurality of inspection assemblies are arranged along an extension direction of the inspection channel. For example, the one or more inspection assemblies may be arranged in different sections of the inspection channel. The inspection assemblies may be arranged closely adjacent to each other or spaced apart from each other, and are distributed in different sections of the inspection channel. As the object under inspection passes through the inspection channel, the object under inspection successively passes through the plurality of inspection assemblies. In such embodiments, each inspection assembly may irradiate the object under inspection from a different viewing angle. For example, when the object under inspection passes through a first inspection assembly, the distributed multi-point source S of the first inspection assembly irradiates the object under inspection from a top side. As the object under inspection continues to move along the inspection channel and passes through a second inspection assembly, the distributed multi-point source S of the second inspection assembly irradiates the object under inspection from a bottom side. As the object under inspection continues to move along the inspection channel and passes through a third inspection assembly, the distributed multi-point source S of the third inspection assembly irradiates the object under inspection from, for example, a left side of the object under inspection. As the object under inspection passes through a fourth inspection assembly, the distributed multi-point source S of the fourth inspection assembly irradiates the object under inspection from, for example, a right side of the object under inspection. A fifth inspection assembly may be provided as needed, and the distributed multi-point source S of the fifth inspection assembly may irradiate the object under inspection from an obliquely upper side of the object under inspection. Additional inspection assemblies may be further provided as needed. Those skilled in the art may determine the appropriate number of inspection assemblies according to the present disclosure, in combination with practical needs and cost considerations. These inspection assemblies may be arranged sequentially along the inspection channel, and adjacent inspection assemblies may be spaced apart from each other at predetermined equal intervals or may be arranged arbitrarily. To acquire more information of the object under inspection, in a transverse direction of the extension direction of the inspection channel, that is, in a circumferential direction of the inspection channel, the one or more inspection assemblies may emit radiation beams toward the inspection channel (the object under inspection therein) from different viewing angles relative to the object under inspection. According to such embodiments, since the plurality of inspection assemblies irradiate the object under inspection from multiple viewing angles relative to the object under inspection, a plurality of cross-sectional images of the object under inspection may be obtained through computer processing. Moreover, as the present disclosure provides the distributed multi-point source S, each inspection assembly may acquire images obtained by irradiating the object under inspection from multiple viewing angles. As a result, CT images of the object under inspection may be obtained without, for example, rotating the object under inspection or rotating the combination of the distributed multi-point source S and the detector DT. Different from CT imaging apparatuses in the related art, gratings are provided in embodiments of the present disclosure, and the CT imaging apparatus provided with gratings may acquire more image information, such as phase-related three-dimensional image information and multi-angle image information of small-angle scattering for the object under inspection. Such image information may be acquired during a single pass of the object under inspection through the inspection channel. By combining these image information, more accurate information regarding tissue identification, structure determination, and the like may be obtained, especially for biological specimens, thereby significantly improving inspection accuracy compared with the use of the absorption image.

In an embodiment, the static CT apparatus includes four inspection assemblies. FIG. 2 shows the arrangement of the four inspection assemblies. In a) of FIG. 2, the distributed multi-point source S irradiates the object under inspection from the top side. In b) of FIG. 2, the distributed multi-point source S irradiates the object under inspection from the left side. In c) of FIG. 2, the distributed multi-point source S irradiates the object under inspection from the bottom side. In d) of FIG. 2, the distributed multi-point source S irradiates the object under inspection from the right side.

In an embodiment, the four inspection assemblies shown in FIG. 2 may be sequentially arranged along the inspection channel, for example, along the extension direction of the inspection channel. The four inspection assemblies may be closely adjacent to each other or may be spaced apart from each other.

The four inspection assemblies enable irradiation on the object under inspection from four directions, namely, top, bottom, left, and right. Through sequential emission by the distributed multi-point sources, omnidirectional irradiation on a stationary object under inspection may be achieved, so as to acquire more data and obtain a three-dimensional image of the object under inspection. In practice, three inspection assemblies may achieve irradiation on a stationary object under inspection from, for example, the top, the bottom, and one lateral side, and the obtained data may be sufficient to reconstruct a three-dimensional image of the object under inspection.

In the foregoing embodiments, it may be approximately regarded that the distributed multi-point source S of each inspection assembly irradiates the corresponding detector DT along a transverse cross-section of the inspection channel. Here, it should be noted that each source of the distributed multi-point source S may emit a conical radiation beam, while the detector DT and the corresponding grating may actually be planar structures. In this case, the so-called irradiation on the corresponding detector DT along the transverse cross-section of the inspection channel means that the radiation beam is distributed within a space having a certain width, while the irradiation direction is generally transverse to the inspection channel. Those skilled in the art may arrange each inspection assembly according to the description of the present disclosure. For example, the four inspection assemblies may irradiate the object under inspection respectively from the top side, the bottom side, the left side, and the right side of the inspection channel. The irradiation directions of the four inspection assemblies are generally a top-to-bottom direction, a bottom-to-top direction, a left-to-right direction, and a right-to-left direction, respectively. However, the radiation beam of each inspection assembly is not planar but a conical beam. In another embodiment, the radiation beam may be fan-shaped. In such embodiments, the distributed multi-point source S may be configured with a corresponding generally linear detector DT.

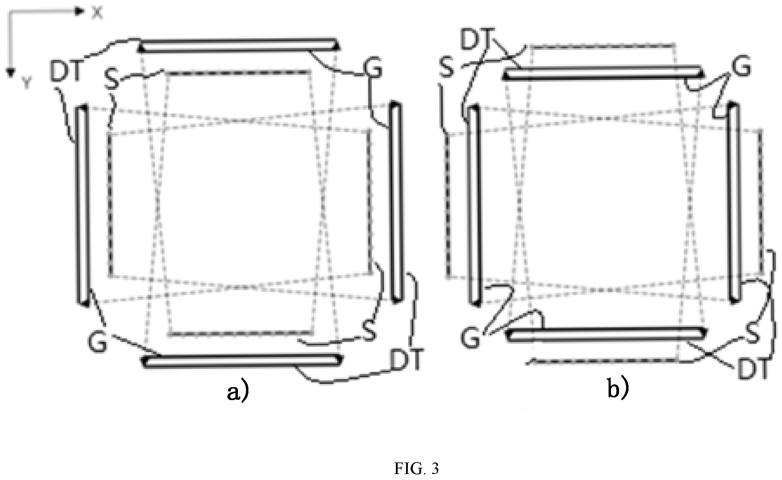

In an embodiment, the one or more inspection assemblies are arranged in a same section of the inspection channel. As shown in FIG. 3, in such embodiments, as the one or more inspection assemblies are arranged in the same section, it is required to prevent the distributed multi-point source S of one inspection assembly from irradiating the distributed multi-point source S of another inspection assembly. In such embodiments, the respective distributed multi-point sources S of the one or more inspection assemblies irradiate the object under inspection from different viewing angles relative to the object under inspection, and the one or more inspection assemblies are arranged such that the directions in which the respective distributed multi-point sources S emit radiation beams form an angle or do not overlap. In other words, for each inspection assembly, the distributed multi-point source S irradiates the corresponding detector DT to define an irradiation plane (generally surface as described above). The plurality of irradiation planes defined by the one or more inspection assemblies do not overlap but instead form an angle, such that the oppositely emitted radiation beams do not interfere with each other. An arrangement example is shown in a) of FIG. 3, and two inspection assemblies are arranged on upper and lower sides, respectively. The detector DT of one inspection assembly and the distributed multi-point source S of the other inspection assembly are offset from each other on the upper side, the corresponding distributed multi-point source S and the corresponding detector DT are offset from each other on the lower side, and their irradiation directions cross each other while remaining offset. The inspection assemblies on the left and right sides may be similarly offset from each other so that the irradiation directions of the two inspection assemblies cross each other while remaining offset. Another arrangement example is shown in b) of FIG. 3, and the detector DT of one inspection assembly and the distributed multi-point source S of the other inspection assembly are offset from each other on the upper side in a manner opposite to that shown in a) of FIG. 3. Other components may be similarly arranged in an opposite manner and will not be described in detail here. According to the arrangements shown in such embodiments, those skilled in the art may conceive of additional arrangements to meet different needs.

In an embodiment, the one or more inspection assemblies are used to operate simultaneously. For example, in an embodiment, the one or more inspection assemblies are arranged in a single section. As the object under inspection passes through the section, the one or more inspection assemblies start operating simultaneously. The respective distributed multi-point sources S start emitting radiation beams to irradiate the object under inspection, and the respective detectors DT simultaneously receive radiation signals passing through the object under inspection and the respective gratings so as to perform inspection. In such embodiments, the object under inspection passes through the one or more inspection assemblies at one time.

In an embodiment, the one or more inspection assemblies are used to operate in a time-sequential manner. For example, in an embodiment, along the extension direction of the inspection channel, the one or more inspection assemblies may be arranged in a plurality of sections of the inspection channel. As the object under inspection moves through these sections, the one or more inspection assemblies are used to start operating sequentially according to a forward movement of the object under inspection.

In embodiments of the present disclosure, for each inspection assembly, the plurality of point sources of the distributed multi-point source S generally emit radiation beams in a time-sequential manner. Since the plurality of point sources of the distributed multi-point source S are arranged along a straight line, the point sources irradiate the object under inspection from different angles. Therefore, a single inspection assembly may perform multi-angle irradiation and acquire images of a plurality of tomographic slices of the object under inspection.

In an embodiment, at least one of the one or more inspection assemblies includes a grating G0. The grating G0 is arranged adjacent to and parallel to the distributed multi-point source S and spaced apart from the distributed multi-point source S by a first distance. Due to the presence of the grating G0, the radiation beams emitted by the distributed multi-point source S are modulated by the grating G0 into one or more interference fringes.

In an embodiment, at least one of the one or more inspection assemblies includes a grating G0 and a grating G1. The grating G1 is arranged adjacent to and parallel to the distributed multi-point source S and spaced apart from the distributed multi-point source S by a second distance different from the first distance. That is, at least one inspection assembly includes two gratings, namely grating G0 and grating G1. In an embodiment, the grating G1 is arranged adjacent to the distributed multi-point source S. In another embodiment, the grating G1 is arranged closer to the detector DT and spaced apart from the detector DT by a third distance.

In an embodiment, at least one inspection assembly includes a grating G2. The grating G2 is arranged adjacent to and parallel to the detector DT and spaced apart from the distributed multi-point source DT by a fourth distance different from the third distance. In an embodiment, at least one inspection assembly includes three gratings, namely grating G0, grating G1, and grating G2. The arrangement of the gratings may refer to the configurations shown in FIG. 1. The gratings G0, G1, and G2 are arranged parallel to each other.

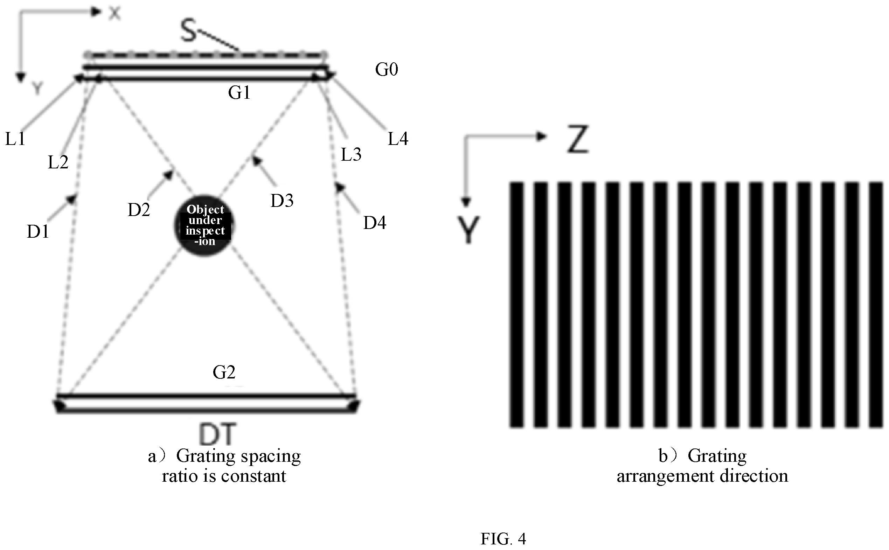

FIG. 4 shows an arrangement of an inspection assembly relative to the object under inspection according to an embodiment of the present disclosure. It should be understood that, according to such embodiments, those skilled in the art may arrange each of the one or more inspection assemblies of the present disclosure as needed. In such embodiments, three gratings G0, G1, and G2 are arranged between the distributed multi-point source S and the detector DT. The three gratings G0, G1, and G2 are arranged parallel to each other and parallel to the detector DT. In FIG. 4, on each radiation path of the radiation beam, a ratio of a spacing L between grating G0 and grating G1 to a spacing D between grating G1 and grating G2 is constant, that is, *L*₁/*D*₁ *= L*₂/*D*₂ *= L*₃/*D*₃ = *L*₄/*D*₄*.*

FIG. 4 further shows an implementation of the grating, for example, in the form of an array of slits. In such embodiments, the periodic structure of the grating includes slits. A periodic direction of the periodic structure may be an arrangement direction of a plurality of slits, while an extension direction of an individual substructure (e.g., a slit) of the periodic structure is perpendicular to the arrangement direction of the plurality of slits. According to the present disclosure, when the extension direction of an individual slit forms a very small angle (for example, relative to being parallel to the inspection channel), such as 10⁻⁴ degree, with the inspection channel (with the extension direction of the inspection channel, i.e., the direction indicated by the movement arrow of the object under inspection W in FIG. 4, namely the Z direction), the interference fringes or moiré fringes generated by the periodic structure formed by the arrangement of the plurality of slits will change significantly. Therefore, in the present disclosure, the requirements of the present disclosure may be satisfied as long as the extension direction of the individual slit is arranged at a very small angle relative to the inspection channel. In an embodiment, as shown in FIG. 4, the irradiation direction of the radiation beam is the Y direction, the extension direction of the inspection channel is the Z direction, and the movement direction of the object under inspection W is the Z direction. For example, the extension direction of an individual slit may be transverse to the extension direction of the inspection channel (i.e., perpendicular to the inspection channel). In such a case, the arrangement direction of the slits (i.e., the periodic direction of the periodic structure) is parallel to the extension direction of the inspection channel, and in FIG. 4, the arrangement direction of the slits extends horizontally from left to right (the Z direction). Here, it should be noted that the irradiation direction of the radiation beam being the Y direction merely indicates the general irradiation direction of the radiation beam. Since the radiation beam may be a conical radiation beam, the irradiation direction of the conical radiation beam being the Y direction does not mean that any ray thereof is strictly aligned with the Y direction; rather, it merely indicates that the conical radiation beam irradiates in the Y direction. Those skilled in the art should understand that, in the configuration shown in FIG. 4, the radiation beam from a point source located on the left side of the distributed multi-point source S may be shaped to irradiate obliquely toward the right in the Y direction, such that a divergence angle thereof covers the detector DT. The radiation beam from a point source located on the right side of the distributed multi-point source S may be shaped to irradiate obliquely toward the left in the Y direction, such that the divergence angle thereof covers the detector DT. Other point sources may be configured in a similar manner such that the divergence angle of the emitted radiation beams enables the radiation beams to cover the detector DT.

According to an aspect of the present disclosure, a CT inspection method is provided, which may be implemented, for example, by the static CT apparatus described above. In such embodiments, the method includes: operating one or more inspection assemblies simultaneously or in a time-sequential manner to inspect the object under inspection from different viewing angles of the inspection channel, where the plurality of point sources of the distributed multi-point source S of each inspection assembly emit radiation beams in a flying-spot manner so as to irradiate the object under inspection from different angles.

In embodiments of the present disclosure, the object under inspection may move through the inspection channel, such that the one or more inspection assemblies may irradiate the object under inspection from different viewing angles. As a result, absorption images, small-angle scattering images, and phase images of different tomographic slices of the object under inspection may be obtained. These images of different tomographic slices may be combined and processed by a computer or processor using an appropriate algorithm to generate a three-dimensional image of at least a portion of the object under inspection. Based on the three-dimensional image, internal constituents, tissues, structures, etc. of the object under inspection may be analyzed to obtain more comprehensive and accurate analysis results.

It should be understood that the various forms of processes shown above may be used, with steps reordered, added, or deleted. For example, the steps described herein may be performed in parallel, sequentially, or in a different order, as long as the desired results of the technical solutions provided by the present disclosure may be achieved, and no limitation is imposed herein.

In the technical solutions of the present disclosure, the collection, storage, use, processing, transmission, provision, disclosure, etc., of user personal information involved all comply with relevant laws and regulations and do not violate public order and good morals.

The foregoing specific embodiments are not intended to limit the scope of protection of the present disclosure. Those skilled in the art will appreciate that various modifications, combinations, sub-combinations, and substitutions may be made to the disclosed embodiments or examples according to design requirements and other factors. Any modification, equivalent substitution, and improvement within the spirit and principle of the present disclosure shall fall within the scope of protection of the present disclosure.

## Claims

1. A static CT apparatus, comprising one or more inspection assemblies,
wherein each of the one or more inspection assemblies comprises:
a distributed multi-point source, comprising a plurality of point sources arranged in an array, and configured to emit radiation beams;
a detector disposed opposite to the distributed multi-point source to receive the radiation beams emitted by the distributed multi-point source, wherein the distributed multi-point source and the detector define an inspection channel; and
a grating arranged between the distributed multi-point source and the detector, wherein the grating is provided with a periodic structure, and the periodic structure is configured to diffract the radiation beams to generate an interference fringe.

2. The static CT apparatus of claim 1, wherein the one or more inspection assemblies are arranged in different sections along an extension direction of the inspection channel.

3. The static CT apparatus of claim 2, wherein in a transverse direction of the extension direction of the inspection channel, the one or more inspection assemblies emit radiation beams toward the inspection channel from different viewing angles in a circumferential direction of the inspection channel relative to an object under inspection.

4. The static CT apparatus of claim 3, wherein the one or more inspection assemblies comprise four inspection assemblies, respective distributed multi-point sources of the four inspection assemblies are respectively arranged on a top side of the inspection channel, a bottom side of the inspection channel, a left side of the inspection channel, and a right side of the inspection channel, so as to emit radiation beams toward the inspection channel respectively from the top side, the bottom side, the left side, and the right side of the inspection channel along a transverse cross-section of the extension direction of the inspection channel.

5. The static CT apparatus of claim 1, wherein the one or more inspection assemblies are arranged in a same section along the extension direction of the inspection channel, and in each of the one or more inspection assemblies, the distributed multi-point source and the detector are oppositely arranged along a transverse direction of the extension direction of the inspection channel, and the one or more inspection assemblies are configured to emit radiation beams toward the inspection channel from different viewing angles in a circumferential direction of the inspection channel.

6. The static CT apparatus of claim 5, wherein the one or more inspection assemblies are arranged such that directions in which respective distributed multi-point sources of the inspection assemblies emit radiation beams toward respective detectors of the inspection assemblies are offset from each other.

7. The static CT apparatus of claim 5, wherein the one or more inspection assemblies comprise four inspection assemblies, respective distributed multi-point sources of the four inspection assemblies are respectively arranged on a top side of the inspection channel, a bottom side of the inspection channel, a left side of the inspection channel, and a right side of the inspection channel, so as to emit radiation beams toward the inspection channel respectively from the top side, the bottom side, the left side, and the right side of the inspection channel along the transverse direction of the extension direction of the inspection channel.

8. The static CT apparatus of claim 1, wherein the one or more inspection assemblies are configured to operate simultaneously or in a time-sequential manner.

9. The static CT apparatus of claim 1, wherein in each of the one or more inspection assemblies, the plurality of point sources of the distributed multi-point source are configured to emit radiation beams in a time-sequential manner.

10. The static CT apparatus of claim 1, wherein in at least one of the one or more inspection assemblies, the gratings comprise:
a first grating arranged adjacent to and parallel to the distributed multi-point source and spaced apart from the distributed multi-point source by a first distance; and/or
a second grating arranged adjacent to and parallel to the distributed multi-point source and spaced apart from the distributed multi-point source by a second distance different from the first distance; or a second grating arranged adjacent to and parallel to the detector and spaced apart from the distributed multi-point source by a third distance; and/or
a third grating arranged adjacent to and parallel to the detector and spaced apart from the distributed multi-point source by a fourth distance different from the third distance.

11. The static CT apparatus of claim 9, wherein in at least one of the one or more inspection assemblies, the gratings comprise a first grating, a second grating, and a third grating, and the first grating, the second grating, and the third grating are parallel to each other.

12. A CT inspection method implemented by the static CT apparatus of claim 1, the method comprising:
operating the one or more inspection assemblies simultaneously or in a time-sequential manner to inspect an object under inspection from different viewing angles of the inspection channel, wherein the plurality of point sources of the distributed multi-point source of each inspection assembly emit the radiation beams in a flying-spot manner so as to irradiate the object under inspection from different angles.
